Europäisches Patentamt

European Patent Office

Office européen des brevets

(19)

(11) **EP 1 230 937 B1**

(12) **FASCICULE DE BREVET EUROPEEN**

(45) Date de publication et mention
de la délivrance du brevet:
**02.11.2006 Bulletin 2006/44**

(51) Int Cl.:
*A61L 15/12* *(2006.01)*     *C08K 3/34* *(2006.01)*

(21) Numéro de dépôt: **01870021.1**

(22) Date de dépôt: **07.02.2001**

(54) **Composition polyurethane thermoplastique à applications orthopediques**

Thermoplastische Formmassen auf Basis von Polyurethan für orthopädische Zwecke

A thermoplastic composition of polyurethane for orthopaedic applications

(84) Etats contractants désignés:
**AT BE CH CY DE DK ES FI FR GB GR IE IT LI LU MC NL PT SE TR**

(43) Date de publication de la demande:
**14.08.2002 Bulletin 2002/33**

(73) Titulaire: **Runlite S.A.**
**4630 Micheroux (BE)**

(72) Inventeurs:
• **Renard, Joseph**
**4607 Mortroux (BE)**

• **Dubois, Philippe**
**4260 Ciplet (BE)**
• **Hick, Christian**
**4880 Aubel (BE)**
• **Vegis, Romuald**
**5060 Tamines (BE)**

(74) Mandataire: **Van Malderen, Michel et al**
**Office van Malderen**
**85/043 Boulevard de la Sauvenière**
**4000 Liège (BE)**

(56) Documents cités:
**US-A- 4 316 457        US-A- 5 717 000**

## Description

### Objet de l'invention

La présente invention se rapporte à un tissu de support flexible imprégné d'une composition de résine thermoplastique à base de polyuréthane polyester et de nanocharges naturelles notamment pour la réalisation de structures orthopédiques telles que bandes, bandages et supports.

L'invention concerne également un tissu imprégné présentant un comportement au feu amélioré pour des applications dans le domaine des décors et parements théâtraux et architecturaux par exemple.

L'invention concerne enfin un procédé de fabrication d'un matériau imprégné pouvant servir à la réalisation de structures orthopédiques.

### Arrière-plan technologique et état de la technique

Un matériau adéquat pour la réalisation de structures orthopédiques doit présenter des caractéristiques telles que la facilité de manipulation (moulage, formage et modelage), la facilité de préparation à leur utilisation ainsi que l'absence de nocivité ou d'incompatibilité pour le membre à soigner et pour la peau en particulier.

Par un traitement préparatoire à leur utilisation, ces matériaux doivent donc acquérir une malléabilité adéquate ainsi qu'un pouvoir adhésif sur eux-mêmes et éventuellement sur d'autres corps. Le traitement envisagé ici se résume à une mise en température, à l'exclusion de l'utilisation de tout solvant ou adhésif externe. Cette mise en température doit permettre l'utilisation de ces matières sans risque de brûlures ou d'autres inconvénients que ce soit pour l'utilisateur ou pour le patient.

La matière employée doit perdre son caractère adhésif une fois qu'elle est revenue à température ambiante et conserver la forme qui lui a été donnée. De plus, elle doit être apte à résister aux contraintes mécaniques dictées par le type d'application précité.

La gamme de température visée est comprise entre 40 et 80°C et peut donc être obtenue de plusieurs manières différentes (bain d'eau chaude, étuve, soufflante à air chaud, etc.).

Idéalement, ces produits sont inertes et ne relarguent pas de substances toxiques, irritantes ou ayant tout autre caractère nocif, de sorte que l'applicateur puisse les utiliser sans moyen de protection particulier.

Ce type de produit ne nécessitera donc, par corollaire, aucun emballage particulier (ex. pochette hermétique).

Les matériaux utilisés jusqu'à présent pour ce type d'application sont le plus souvent formés à partir de polymères linéaires d'ester cyclique, caractérisés par les fonctions "-COO-" séparées par des radicaux méthylènes comprenant de 2 à 7 carbones, comme par exemple les polymères de la 2-oxepanone, mieux connu sous le nom de polycaprolactone.

Le brevet US-A-3 692 023 propose l'utilisation de ce polymère pour l'obtention d'un matériel de contention. Par ailleurs, le brevet US-A-4 273 115 décrit un matériel de contention cylindrique basé sur un tricot Raschel particulier imprégné de polycaprolactone de poids moléculaire 40 000 environ. Dans le brevet US-A-4 316 457, on propose la préparation d'un polyuréthane thermoplastique linéaire d'un poids moléculaire de 40 000 à 45 000. Il s'agit d'une imprégnation réactive où un prépolymère formé par polycondensation d'un diisocyanate et d'un polyester diol, tel que la polycaprolactone par exemple, est mélangé à un polymère de la famille des polyesters diol avant imprégnation d'un tricot Raschel. Tels que décrits, ces matériaux deviennent fluides à température de fusion et ont une adhésivité mal contrôlée.

Pour améliorer ces matériaux, diverses méthodes ont été utilisées, comme, tout d'abord, des moyens externes tels que séparateurs en polyéthylène cités dans les brevets américains US-A-4 273 115 et US-A-4 316 457.

D'autres moyens de résoudre ces problèmes ont été proposés et reposent sur des adaptations physiques internes au matériau formé à partir de ces polyesters. La viscosité de ces polyesters à l'état fondu peut être diminuée par l'ajout d'une grande quantité de charge minérale, ce qui entraîne également une diminution de l'adhésivité du matériau comme le suggère la demande de brevet européen EP-A-0 169 037.

Le problème lié à la trop grande fluidité des polycaprolactones et des polyuréthanes linéaires s'y rapportant peut être en partie résolu, comme dans les brevets US-A-4 273 115 et US-A-4 316 457, par la présence d'un support de type tricot Raschel.

Une autre solution proposée afin de pouvoir moduler la viscosité et l'adhésivité de ces produits est le recours à la réticulation chimique. Cette technique a été développée dans les documents US-A-4 240 415 et EP-A-0 235 500. De manière similaire, une autre solution proposée est la préparation d'un matériau à partir d'un réseau polymère interpénétré ou semi-interpénétré, impliquant donc une réticulation chimique, c'est-à-dire le pontage des chaînes polymères par des liaisons chimiques covalentes. Ce procédé est décrit dans le brevet US-A-5 652 053. Une combinaison est obtenue par le mélange de deux polymères, le premier pouvant appartenir à la famille des polymères et copolymères vinyliques, d'ester acrylique ou méthacrylique mais aussi à la famille des polyurées et polyuréthanes, notamment à partir d'une polycaprolactone diol et d'un diisocyanate. Le second constituant peut être de nature polyéther ou polyuréthane linéaire ou réticulé, comme par exemple un polyuréthane formé à partir de polycaprolactone diol, polycaprolactone triol et de diisocyanate. Ce procédé permet donc d'obtenir un matériau semi-réticulé chimiquement.

## Buts de l'invention

La présente invention vise à fournir une solution ne présentant pas les inconvénients de l'état de la technique.

En particulier, l'invention a pour but de fournir une solution pour moduler la viscosité et l'adhésivité des produits à base de polyuréthane polyester, obtenu plus particulièrement par polycondensation de polycaprolactone diol et de diisocyanate, qui sont utilisés en particulier pour des applications de contention, sans nécessiter la présence d'un support mécanique tel qu'un tissu Raschel ou sans le recours à la réticulation chimique de la résine polymère.

Un but complémentaire de l'invention est de produire une résine polyuréthane modifiée, dont le comportement au feu est nettement amélioré par rapport à la résine de base.

## Principaux éléments caractéristiques de l'invention

La présente invention concerne un tissu de support flexible imprégné d'un matériau nanocomposite, formé à partir de résine de polyuréthane thermoplastique semi-cristalline et de précurseurs de nanocharges, caractérisé en ce que lesdites nanocharges présentent des couches silicates organo-modifiées en surface et sont dispersées dans ladite résine par intercalation et/ou exfoliation des différentes couches, lesdites nanocharges constituant des ponts de réticulation physique entre les chaînes polymères de ladite résine thermoplastique.

Avantageusement, ladite résine thermoplastique est obtenue par mélange en présence d'un catalyseur d'une première solution organique de polymère et d'une seconde solution organique de prépolymère, le prépolymère (PP) étant un polymère diisocyanate obtenu à partir de la réaction d'un oligomère bifonctionnel avec un excès molaire de réactif diisocyanate et le polymère (P) étant un polymère bifonctionnel allongeur de chaîne. L'oligomère bifonctionnel comporte des fonctions terminales sélectionnées parmi le groupe constitué par -OH, -NH$_2$, - COOH et -SH, de préférence des diols de faible masse moléculaire.

De préférence, l'oligomère bifonctionnel est une polycaprolactone diol de masse moléculaire moyenne en nombre comprise entre 1000 et 10000, et encore de préférence entre 2000 et 6000.

Avantageusement, le réactif diisocyanate est sélectionné parmi le groupe des diisocyanates aromatiques, aliphatiques et cycloaliphatiques et est de préférence l'hexaméthylène diisocyanate ou le 4,4'-dicyclohexylméthane diisocyanate. Le polymère bifonctionnel allongeur de chaîne est de préférence une polycaprolactone de faible masse moléculaire.

Toujours avantageusement, le catalyseur est une amine tertiaire ou un composé à base d'étain, de préférence le dilaurate de dibutyl-étain.

Selon une caractéristique de l'invention, les nanocharges à couches silicates organo-modifiées en surface présentent une substitution des ions sodium par des ions ammonium porteurs de chaînes alkyles, de préférence l'octadécylammonium quaternisé, le diméthyldioctadécylammonium, le (2 éthyl-hexyl)diméthyl(suif hydrogéné)ammonium, le (2-hydroxyéthyl)méthyl(suif hydrogéné)ammonium.

De préférence, lesdites nanocharges sont constituées de montmorillonite organo-modifiée par des cations (2 éthyl-hexyl)diméthyl(suif hydrogéné)ammonium ou (2-hydroxyéthyl)méthyl(suif hydrogéné)ammonium ou un mélange des deux.

De manière particulièrement avantageuse, le matériau nanocomposite imprégnant le tissu de l'invention présente un taux de chargement en nanocharges compris entre 0.1 et 20% en poids, et de préférence entre 2 et 15% en poids. Il contient également des microcharges, de préférence le borate de zinc, le dihydroxyde de magnésium ou le trihydrate d'aluminium, le taux de chargement en microcharges étant compris entre 5 et 50% en poids, de préférence entre 10 et 30% en poids.

Selon une autre caractéristique, le matériau nanocomposite imprégnant le tissu de l'invention présente des propriétés anti-feu qualitativement améliorées par rapport à un matériau similaire ne comportant pas de nanocharges organo-modifiées ou comportant des nanocharges non organo-modifiées. En particulier, ledit matériau se consume sans formation de gouttes incandescentes lorsqu'il est mis au contact d'une flamme.

D'un point de vue chimique, la présente invention a pour objet de proposer un matériau nanocomposite, caractérisé par le fait que lesdites nanocharges, organomodifiées par des ions ammonium porteurs de fonctions capables de réagir avec la fonction isocyanate, réagissent en surface avec des chaînes polyuréthane par les extrémités isocyanates desdites chaînes et pontent ainsi lesdites chaînes entre elles.

Un autre aspect de la présente invention concerne un procédé, de préférence pour l'obtention d'une structure orthopédique, comprenant l'imprégnation d'un tissu de support flexible, avec une composition comprenant un prépolymère (PP) comportant deux groupes terminaux isocyanante, un polymère bifonctionnel (P) allongeur de chaîne, des précurseurs de nanocharges organo-modifiées, au moins un catalyseur et un solvant, caractérisé par les étapes suivantes :

-   la préparation d'une solution de prépolymère (PP) diisocyanante dans un solvant organique, à partir de la réaction d'un oligomère bifonctionnel avec un excès molaire de diisocyanate ;
-   la préparation d'une solution organique de polymère bifonctionnel (P) allongeur de chaîne, contenant également lesdites nanocharges organo-modifiées et un catalyseur ;
-   l'imprégnation dudit tissu de support flexible par trempes successives dans un mélange des deux dites solutions ;

- l'élimination du solvant par mise en température selon une rampe de chauffe allant de la température ambiante jusqu'à 150°C ;
- le retour à la température ambiante pour l'obtention d'un produit final de ladite structure orthopédique.

Le matériau nanocomposite de l'invention sert à la confection d'une structure orthopédique obtenue par imprégnation d'un tissu de support avec ce matériau. Le tissu de support est un tissu flexible à larges mailles, de préférence tissé ou tricoté, en matière cellulosique, de préférence en coton, ou synthétique, encore de préférence en acrylate ou nylon, et dont la plus petite dimension des ouvertures est comprise entre 0,142 et 1,613 cm$^2$, de préférence égale à 0,323 cm$^2$.

Le tissu imprégné de l'invention peut aussi convenir comme matériau résistant au feu dans le domaine des décors et parements théâtraux et architecturaux.

## Brève description des figures

La figure 1 représente un exemple de test qualitatif au feu pour une imprégnation de trame avec une résine polymère sans nanocharges.

La figure 2 représente un exemple de test qualitatif au feu pour une imprégnation de trame avec une résine polymère avec nanocharges, selon l'invention.

## Description détaillée de l'invention

La présente invention repose sur la fabrication d'un tissu imprégné d'un matériau composite constitué d'une résine de polyuréthane thermoplastique semi-cristalline chargée de nanocharges à couches silicates organo-modifiées en surface et dispersées dans la résine suite à une intercalation et/ou une exfoliation des différentes couches. Dans la présente demande, cette résine comportant des nanocharges sera désignée par le terme nanocomposite ou encore nanomatériau.

Le nanocomposite est produit en cours d'imprégnation d'un tissu de support. La réticulation est cette fois de nature physique, les nanocharges jouant le rôle de ponts de réticulation et/ou de branchements physiques entre les chaînes polymères de la résine thermoplastique. Les différentes couches silicates exfoliées forment un réseau qui interagit avec le réseau de chaînes polymères de la résine, en pontant lesdites chaînes.

Ce principe confère au matériau des propriétés thermomécaniques, d'adhésion et de surface modulables selon la nature et les quantités de nanocharge organophile ajoutées, mais aussi de meilleures propriétés au niveau du comportement ignifuge du matériau ouvrant ainsi une application possible dans le domaine des décors et parements par exemple.

L'invention se rapporte donc en particulier à un procédé de fabrication pour la réalisation d'un matériau, pouvant servir à la réalisation de structures orthopédiques, qui comprend l'imprégnation d'un tissu de support, tel que par exemple une trame de coton tissé à large maillage, par une solution de polymère, ainsi que l'élimination du solvant du support imprégné.

Le matériau support est de préférence un tissu flexible à mailles larges, de préférence tricoté, qui se caractérise par un réseau d'ouvertures relativement larges. La plus petite dimension des ouvertures peut généralement être d'au moins 0,097 cm$^2$, de préférence au minimum de 0,142 cm$^2$, environ de 0,219 cm$^2$ et généralement ne dépassant pas 1,613 cm$^2$, tout en étant encore de préférence égale à 0,323 cm$^2$. Les ouvertures peuvent présenter n'importe quelle configuration, par exemple carrée, polygonale ou similaire. L'ouverture devra être assez large pour que, dans le produit fini, la composition de polymère ne forme pas de préférence des sites imperméables à l'air sur l'étendue des ouvertures. Les matières pouvant être utilisées comprennent les matières cellulosiques, telles que le coton, les matières synthétiques, telles que les acrylates et le nylon, ou encore la combinaison de celles-ci.

La solution polymère est formée à partir d'un prépolymère polyester de "type polyuréthane" comportant deux groupes terminaux isocyanates obtenus par la réaction d'un composé bifonctionnel réactif vis-à-vis d'un groupe isocyanate avec un excès molaire en isocyanate en présence d'un catalyseur. Cette solution contient également les nanocharges nécessaires à la formation du nanomatériau.

La réalisation de cette résine se déroule en deux étapes. La première étape fait réagir un diisocyanate présentant deux groupes terminaux isocyanate et un diol pour arriver à un prépolymère présentant deux groupes terminaux isocyanate. La seconde étape comprend l'allongement de chaîne du prépolymère au moyen d'un polymère bifonctionnel en présence d'un catalyseur qui peut être une amine tertiaire ou un composé à base d'étain, tel que le dilaurate de dibutyl-étain.

Le diisocyanate qui est utilisé pour la première étape peut être choisi parmi les diisocyanates aromatiques, aliphatiques ou cycloaliphatiques qui sont habituellement utilisés pour former les polyuréthanes. Néanmoins, les diisocyanates aliphatiques ou cycloaliphatiques sont préférés. C'est par exemple le cas de l'hexaméthylène diisocyanate ou le 4,4'-dicyclohexylméthane diisocyanate.

Le composé bifonctionnel utilisé comme produit de départ pour la préparation du prépolymère peut être un oligomère comportant des groupes terminaux réactifs tels que des groupes -OH, -NH$_2$, -COOH ou -SH. Les composés préférés étant des diols de faible masse moléculaire tels que la polycaprolactone diol de masse moléculaire moyenne en nombre comprise entre 1 000 et 10 000, et de préférence de 2000 à 6000.

Les nanocharges en suspension dans le prépolymère sont des charges naturelles telles que des argiles à couches silicates comme la montmorillonite ou l'hectorite, dont l'une des trois dimensions de couche est de l'ordre du nanomètre. Le facteur de forme, qui est le rapport de la plus grande dimension sur la plus petite, cette dernière étant de l'ordre du nanomètre, est généralement compris entre 100 et 1 000. Ces charges sont organo-modifiées en surface suite à une réaction de substitution cationique des ions Na$^+$ par des ions ammonium porteurs de chaînes alkyles comme par exemple l'octadécylammonium quaternisée, le diméthyldioctadécylammonium, le cation 2 (éthyl-hexyl)diméthyl(suif hydrogéné)ammonium, le cation bis (2-hydroxyéthyl)méthyl(suif hydrogéné)ammonium.

Selon la nature de ces chaînes alkyles, une intercalation et/ou une exfoliation peut s'opérer lors de la synthèse du matériau individualisant les nanocouches dispersées dans le polyuréthane.

De plus, une réticulation physique peut s'opérer par suite de l'enchevêtrement des chaînes polymères polyuréthanes et de leurs interactions avec la surface organo-modifiée des nanocharges. Il en résulte la formation d'un réseau tridimensionnel au sein duquel le polyuréthane est réticulé par interaction/greffage en surface des nanocouches organophiles, ces nanocharges jouant le rôle d'agents de réticulation physique. Ce réseau confère au matériau final des propriétés mécaniques spécifiques.

En outre, selon le taux de chargement en nanocharges, qui est compris entre 0,1 et 20% en poids, préférentiellement entre 3 et 15% en poids, ainsi qu'en mélangeant les différents types de particules, on peut moduler les propriétés physico-mécaniques (rigidité, élasticité), d'adhésion et de propriétés de surface (rugosité) des matériaux.

Une propriété importante de ces nanocomposites est un comportement au feu différent et amélioré de celui connu jusqu'à présent pour de telles compositions. Il est remarquable et surprenant de constater que la présence des nanocharges, en plus de leur rôle sur les propriétés physico-mécaniques, d'adhésion et de surface, confère à la composition la propriété de ne plus former de gouttes incandescentes lorsque le produit est mis au contact d'une flamme, mais de se consumer en formant une trame résiduelle intumescente et carbonisée. Ce comportement au feu peut également être amélioré par l'ajout complémentaire de microcharges telles que le borate de zinc, le dihydroxyde de magnésium et le trihydrate d'aluminium à hauteur de 5 à 50% en poids , de préférence de 10 à 30% en poids.

En résumé, un phénomène important remarqué pour les composés usuels est le fait qu'une résine thermoplastique telle que celle décrite dans les brevets US-A-4 273 115 et US-A-4 316 457 brûle en formant des gouttelettes enflammées, ce qui entraîne une propagation du feu autour du matériau. La présente invention vise donc également à la réalisation d'une résine au sein de laquelle la dispersion d'une nanocharge entraîne une intercalation et/ou une exfoliation permettant d'obtenir une résine modifiée, dont le comportement au feu est nettement amélioré, présentant un temps d'inflammation supérieur et ne coulant plus lorsqu'elle brûle. Par l'ajout de ces nanocharges, on obtient également une amélioration des propriétés de surface, la rugosité du matériau diminuant avec l'ajout de ces particules.

## Description d'une forme d'exécution préférée de l'invention

On détaillera ci-après un certains nombre d'exemples de mise en oeuvre préférentielle de l'invention.

Dans la réalisation de ces essais, on procède en deux étapes : on prépare d'abord les deux constituants, le polymère (P) et le prépolymère (PP) et ensuite on les mélange dans des quantités précises afin de respecter la stoechiométrie de la réaction. Une fois mélangée, la résine vient imprégner le support de coton par trempes successives et la polymérisation d'extension de chaînes et d'évaporation du solvant s'opèrent par élévation de température.

Dans la description des exemples, on détaillera la composition de P et PP ainsi que les quantités de PP ajouté à la solution de P.

Procédure de caractérisation

### 1. Taux de chargement en résine

La méthode utilisée comprend la mise en solution de la résine dans le chloroforme.

Un échantillon imprégné de 15 cm sur 7 cm est découpé en petits carrés d'environ 3 mailles sur 3 afin de faciliter la mise en solution. Ces morceaux sont pesés afin de déterminer ultérieurement le taux de chargement. On les place dans un bécher de 250 ml contenant 150 ml de CHCl$_3$. On laisse sous agitation magnétique pendant une nuit. On vérifie si la dissolution de la résine est complète ou s'il s'opère un gonflement. Enfin, on filtre la solution sur buchner et on récupère le coton ainsi que la solution.

#### *Solution*

On lave la solution pour éliminer le catalyseur résiduel :

- Placer la solution dans une ampoule à décanter
- Laver avec 150 ml de HCl 0,1 M
- Laver deux fois avec 150 ml de H$_2$O

On rotavape la solution.

On récupère la résine pour effectuer les différentes analyses telles que l'analyse des masses moléculaires.

#### *Coton*

Le coton une fois récupéré est séché à l'étuve pendant une nuit à la température de 110°C pour être pesé.

Le taux de chargement peut donc être calculé par différence.

### 2. Test de traction

On découpe un rectangle de 20 mailles de long sur 15 mailles de large. Dans ce rectangle, on découpe ensuite deux encoches de manière à obtenir une géométrie de type « os de chien ». La partie centrale a les dimensions de 4 mailles de large sur 11 mailles de haut.

Pour chaque caractérisation, 5 éprouvettes sont nécessaires. Une fois ces éprouvettes découpées, elles sont mises en forme afin de rentrer dans les pinces multibroches prévues pour les tests de traction. Pour ce faire, on chauffe dans de l'eau à 70°C les parties supérieure et inférieure des éprouvettes. On place ensuite ces parties dans les pinces et on les met bien en forme. Une fois cette préparation terminée, les tests peuvent être effectués.

### Déroulement des tests

L'éprouvette est placée dans le banc de traction, les pinces étant refermées et bien fixées au banc de traction. La vitesse d'écartement des pinces est de 50 mm/minute. La température est de 24-25°C. Une précharge de 5N est appliquée. Cette précharge sert à éviter toute erreur due à un mauvais placement de l'éprouvette et à être certain de commencer le test dans les mêmes conditions initiales pour chaque éprouvette. On établit une courbe expérimentale charge-élongation. A partir de cette courbe, on obtient deux résultats : la résistance par contrainte à la rupture, $\sigma$ exprimé en N, et la rigidité exprimée par le module d'élasticité, ce qui équivaut à la plus grande pente de la courbe, E.

### 3. Test de compression

On découpe une bande d'échantillon imprégné de 40 mailles de haut sur 25 mailles de large, la longueur étant prise dans le sens de la production. On chauffe cette bande dans de l'eau à 70°C pendant deux minutes. Une fois chaude, la bande est enroulée sur un cylindre de 4,5 cm de diamètre. La taille de la bande permet de réaliser exactement deux tours de ce cylindre, les extrémités de la bande se superposant. Pour chaque caractérisation, le test est réalisé sur 5 cylindres. Le cylindre est placé entre les deux plateaux du banc de compression, l'endroit de superposition des extrémités étant situé sur le plateau inférieur de manière à éviter un point dur. La vitesse de compression est de 50 mm/min. Une précharge de 5 N est appliquée afin d'éliminer toute erreur de mauvais positionnement du cylindre. Le test est effectué jusqu'à une compression de 35 mm. Pour chaque test, on mesure la charge pour une compression de 22,5 mm ($\sigma_c$ exprimé en N) et le module de compression, représenté par la plus forte pente ($E_c$ exprimé en N/mm).

### Exemple 1 : imprégnation sans nanocharges

Dans cet exemple, l'imprégnation de la trame par la résine polymère se déroule en l'absence de toute charge. Cet exemple servira de référence et permettra de comparer les performances des matériaux obtenus en présence de nanocharges tels que repris dans les exemples suivants.

- Préparation de P :

  Dans un bécher, on dissout 200 g de polycaprolactone diol de masse moléculaire 4 000 dans 200 g de dichlorométhane et on y ajoute 0,13 g de DABCO comme catalyseur.

- Préparation de PP :

  Dans un bécher, on mélange 50 g de polycaprolactone diol de masse moléculaire 4 000 avec 50 g d'hexaméthylène diisocyanate dans 100 g de dichlorométhane.

- Mélange de P et PP :

  Dans le bécher contenant P, on ajoute 35,11 g de la solution PP. On verse le tout dans un récipient en aluminium permettant l'imprégnation du coton par trempes successives. On utilise des échantillons de 90 mm x 230 mm, présentant un repère situé à 190 mm du bord inférieur, lorsque l'échantillon est tenu verticalement. On place ensuite ces échantillons de coton imprégné dans une étuve chauffée selon une rampe de température allant de la température ambiante à 150°C. Le produit final est obtenu dès retour à la température ambiante.

Le pourcentage de résine par rapport au coton (DRC) est déterminé en effectuant le rapport entre la masse de résine réelle (masse de l'échantillon imprégné moins la masse de coton vierge) et la masse de l'échantillon imprégné. Si X est la masse de l'échantillon imprégné et Y la masse de coton vierge, c'est-à-dire séché et non imprégné, on a :

$$DRC = ((X-Y)/X)*100$$

Pour ce type d'échantillon, le degré d'imprégnation est de l'ordre de 80%. Les masses moléculaires moyennes en nombre et en masse sont également déterminées par analyse chromatographique par perméation de gel. Les résultats suivants ont été obtenus :
Mn = 23 300, Mw = 39 000, Mw/Mn = 1,85.
L'analyse calorimétrique différentielle des températures caractéristiques donne la température de transition vitreuse et la température de fusion. Ces deux températures sont :

Tg = -63,45°C, Tm = 49,75°C.

Des tests de solubilisation ont également été effectués, ces échantillons sont parfaitement solubles dans des solvants tels que le dichlorométhane ou le tétrahydrofurane.

Au niveau des propriétés mécaniques, on obtient pour les tests de traction les résultats suivants :

Module d'élasticité : $E_t$ = 70,57 N/mm

Charge maximale : $\sigma_t$ = 509,19 N

En ce qui concerne les tests de compression, on obtient :

Module de compression : $E_c$ = 35,88 N/mm
Charge pour une compression de 22,5 mm : $\sigma_c$ = 366,19 N

Au niveau des propriétés au toucher, le matériau présente un aspect relativement rugueux.

Les résultats des propriétés anti-feu réalisées selon une méthode analogue à celle décrite dans la norme « DIN 4102 Teil 1, Kleinbrenner B2 », on obtient les résultats suivants :

Temps d'inflammation : 7 secondes
Hauteur maximum de la flamme pendant les 20 sec après 20 sec : > 15 cm
Le repère est atteint.

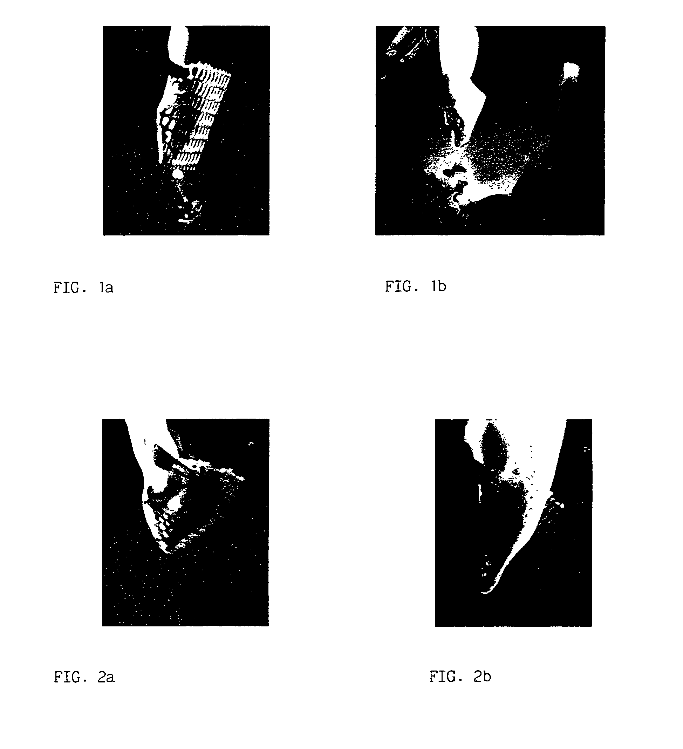

De plus on observe, à la figure 1, la formation de gouttes enflammées capables d'enflammer à leur tour une feuille de papier positionnée en dessous du matériau testé.

### Exemple 2 : imprégnation avec la montmorillonite (Na$^{\pm}$) non organo-modifiée

• Préparation de P :

Dans un bécher, on dissout 200 g de polycaprolactone diol de masse moléculaire 4 000 dans 200 g de dichlorométhane. On ajoute à cette solution 21,77 g de montmorillonite Na$^+$ (surface non organo-modifiée), soit 10% en poids de charge par rapport au total de résine polycaprolactone, ainsi que 0,13 g de DABCO.

• Préparation de PP :

Dans un bécher, on mélange 50 g de polycaprolactone diol de masse moléculaire 4 000 avec 50 g d'hexaméthylène diisocyanate dans 100 g de dichlorométhane.

• Mélange de P et PP :

Dans le bécher contenant P, on ajoute 35,11 g de la solution PP. On verse le tout dans un récipient en aluminium permettant l'imprégnation du coton par trempes successives. On place ensuite ces échantillons de coton imprégné dans une étuve chauffée selon la même rampe de température que dans l'exemple précédent.

Le produit final est obtenu une fois redescendu à la température ambiante. Pour ce type d'échantillon, le degré d'imprégnation moyen (DRC) est de 81%.

Les masses moléculaires moyennes en nombre et en masse sont également déterminées par analyse chromatographique par perméation de gel. Les résultats suivants ont été obtenus :

Mn = 16 300, Mw = 31 700, Mw/Mn = 1,94.

L'analyse calorimétrique différentielle des températures caractéristiques donne la température de transition vitreuse et la température de fusion. Ces deux températures sont :

Tg = -63,98°C, Tm = 51,54°C. Des tests de solubilisation ont également été effectués, ces échantillons sont parfaitement solubles dans des solvants tels que le dichlorométhane ou le tétrahydrofurane.

Au niveau des propriétés mécaniques, on obtient pour les tests de traction les résultats suivants :

Module d'élasticité : $E_t$ = 95,66 N/mm

Charge maximale : $\sigma_t$ = 733,60 N

En ce qui concerne les tests de compression, on obtient :

Module de compression : $E_c$ = 53,25 N/mm
Charge pour une compression de 22,5 mm : $\sigma_c$ = 495,04 N

Au niveau de la rugosité, par contre, aucune modification n'est observée, le matériau étant toujours aussi rugueux.

Les résultats des propriétés anti-feu, mesurées selon une méthode analogue à celle décrite dans la norme « DIN 4102 Teil 1, Kleinbrenner B2 », sont les suivants :

Temps d'inflammation : 8 secondes
Hauteur maximum de la flamme pendant les 20 sec après 20 sec : 9 cm

Le repère est atteint.

De manière similaire à la composition de l'exemple n°1, ce produit présente la formation de gouttes enflammées, preuve de la non-réalisation d'un nanocomposite, sans intercalation ni exfoliation, comme en témoigne l'analyse par diffraction de rayons X, démontrant la non-intercalation des couches silicates par le polymère.

**Exemple 3 : imprégnation avec la montmorillonite organo-modifiée par des cations (2 éthyl-hexyl)diméthyl(suif hydrogéné)ammonium**

- Préparation de P :

  Dans un bécher, on dissout 200 g de polycaprolactone diol de masse moléculaire 4 000 dans 200 g de dichlorométhane. On ajoute à cette solution 21,77 g de montmorillonite organo-modifiée par des cations (2 éthyl-hexyl) diméthyl (suif hydrogéné) ammonium , soit 10% en poids de charge par rapport au total de résine polycaprolactone, ainsi que 0,13 g de DABCO.

- Préparation de PP :

  Dans un bécher, on mélange 50 g de polycaprolactone diol de masse moléculaire 4 000 avec 50 g d'hexaméthylène diisocyanate dans 100 g de dichlorométhane.

- Mélange de P et PP :

  Dans le bécher contenant P, on ajoute 35,11 g de la solution PP. On verse le tout dans un récipient en aluminium permettant l'imprégnation du coton par trempes successives. On place ensuite ces échantillons de coton imprégné dans une étuve chauffée selon une rampe de température allant de la température ambiante à 150°C. Le produit final est obtenu dès retour à la température ambiante.

Pour ce type d'échantillon, le degré d'imprégnation moyen (DRC) est de 81%. Les masses moléculaires moyennes en nombre et en masse sont également déterminées par analyse chromatographique par perméation de gel. Les résultats suivants ont été obtenus :
Mn = 10 800, Mw = 19 700, Mw/Mn = 1,82.
L'analyse calorimétrique différentielle des températures caractéristiques donne la température de transition vitreuse et la température de fusion. Ces deux températures sont :
Tg = -64,20°C, Tm = 51,36°C.
Des tests de solubilisation ont également été effectués, ces échantillons sont parfaitement solubles dans des solvants tels que le dichlorométhane ou le tétrahydrofurane.
Au niveau des propriétés mécaniques, on obtient pour les tests de traction les résultats suivants :

  Module d'élasticité : $E_t$ = 52,85 N/mm
  Charge maximale : $\sigma_t$ = 643,90 N

Par rapport au matériau réalisé sans apport de charges, on obtient des propriétés à la traction correctes malgré des masses moléculaires relativement faibles.
En ce qui concerne les tests de compression, on obtient :

  Module de compression : $E_c$ = 39,44 N/mm
  Charge pour une compression de 22,5 mm : $\sigma_c$ = 367,72 N

De plus, le nouveau matériau présente un aspect nettement moins rugueux au toucher. Les propriétés anti-feu mesurées selon les mêmes critères que précédemment montrent également une amélioration indiquée par les résultats suivants :

  Temps d'inflammation : 14 secondes
  Hauteur maximum de la flamme pendant les 20 sec après 20 sec : 3 cm

Le repère est atteint.
Ce matériau ne forme plus de gouttes enflammées comme dans les exemples 1 et 2 (voir figure 2) et ne propage donc plus le feu à la feuille de papier positionnée en dessous du matériau.

**Exemple 4 : imprégnation avec la montmorillonite organo-modifiée par des cations bis(2-hydroxyéthyl)méthyl(suif hydrogéné)ammonium**

- Préparation de P :

  Dans un bécher, on dissout 200 g de polycaprolactone diol de masse moléculaire 4 000 dans 200 g de dichlorométhane. On ajoute à cette solution 21,77 g de montmorillonite organo-modifiée par des cations bis(2-hydroxyéthyl)méthyl (suif hydrogéné)ammonium , soit 10% en poids de charge par rapport au total de résine polycaprolactone, ainsi que 0,13 g de DABCO.

- Préparation de PP :

  Dans un bécher, on mélange 50 g de polycaprolactone diol de masse moléculaire 4 000 avec 50 g d'hexaméthylène diisocyanate dans 100 g de dichlorométhane.

- Mélange de P et PP :

  Dans le bécher contenant P, on ajoute 43,36 g de la solution PP. On verse le tout dans un récipient en aluminium permettant l'imprégnation du coton par trempes successives. On place ensuite ces échantillons de coton imprégné dans une étuve chauffée selon une rampe de température allant de la température ambiante à 150°C (voir exemples précédents). Le produit final est obtenu dès retour à la température ambiante.

Pour ce type d'échantillon, le degré d'imprégnation moyen (DRC) est de 81%. Les masses moléculaires moyennes en nombre et en masse sont également déterminées par analyse chromatographique par perméation de gel. Les résultats suivants ont été obtenus :

Mn = 21 500, Mw = 38 600, Mw/Mn = 1,80.

L'analyse calorimétrique différentielle des températures caractéristiques donne la température de transition vitreuse et la température de fusion. Ces deux températures sont :

Tg = -63,72°C, Tm = 51,18°C.

Des tests de solubilisation ont également été effectués. La mise en solution de ces échantillons s'est avérée très délicate, le filtre nécessaire à la séparation de la résine du coton se bouchant. Ce phénomène peut s'expliquer par la formation d'un système réticulé physiquement par suite de la réaction avec des fonctions alcool disponibles au niveau des chaînes alkyles des ions ammonium. Au niveau des propriétés mécaniques, on obtient pour les tests de traction les résultats suivants :

Module d'élasticité : $E_t$ = 45,15 N/mm
Charge maximale : $\sigma_t$ = 388,23 N

En ce qui concerne les tests de compression, on obtient :

Module de compression : $E_c$ = 41,53 N/mm
Charge pour une compression de 22,5 mm : $\sigma_c$ = 436,81 N

Au niveau de la compression, on améliore la charge maximale nécessaire pour obtenir un écrasement de 22,5 mm. Le matériau est donc plus ductile, comme le montre le test à la traction, tout en ayant une résistance à la compression améliorée par rapport à l'exemple 1. De nouveau, le matériau ne forme pas de gouttes enflammées, ce qui traduit un comportement similaire à celui de l'exemple 3. L'analyse par diffraction de rayons X témoigne de la formation d'un matériau nanocomposite, avec intercalation et exfoliation, au moins partielle, des couches silicates.

## Exemple 5 : imprégnation avec le borate de zinc

- Préparation de P :

  Dans un bécher, on dissout 200 g de polycaprolactone diol de masse moléculaire 4 000 dans 200 g de dichlorométhane. On ajoute à cette solution 43,54 g de borate de zinc, soit 20% en poids de charge par rapport au total de résine polycaprolactone, ainsi que 0,13 g de DABCO.

- Préparation de PP :

  Dans un bécher, on mélange 50 g de polycaprolactone diol de masse moléculaire 4 000 avec 50 g d'hexaméthylène diisocyanate dans 100 g de dichlorométhane.

- Mélange de P et PP :

  Dans le bécher contenant P, on ajoute 35,11 g de la solution PP. On verse le tout dans un récipient en aluminium permettant l'imprégnation du coton par trempes successives. On place ensuite ces échantillons de coton imprégné dans une étuve chauffée selon une rampe de température allant de la température ambiante à 150°C (voir exemples précédents). Le produit final est obtenu dès retour à la température ambiante.

Pour ce type d'échantillon, le degré d'imprégnation moyen (DRC) est de 79%. Les masses moléculaires moyennes en nombre et en masse sont également déterminées par analyse chromatographique par perméation de gel. Les résultats suivants ont été obtenus :

Mn = 20 900, Mw = 38 600, Mw/Mn = 1,76.

L'analyse calorimétrique différentielle des températures caractéristiques donne la température de transition vitreuse et la température de fusion. Ces deux températures sont :

Tg = -64,47°C, Tm = 51,00°C.

Au niveau des propriétés mécaniques, on obtient pour les tests de traction les résultats suivants :

Module d'élasticité : $E_t$ = 71,29 N/mm
Charge maximale : $\sigma_t$ = 659,16 N

Ces résultats sont semblables à ceux obtenus pour les échantillons réalisés sans adjonction de charges, en ce qui concerne le module d'élasticité, avec toutefois une amélioration au niveau de la charge maximale supportée. En ce qui concerne la rugosité de surface, le matériau se présente comme lorsqu'il y a adjonction de charges ne formant pas de nanocomposite telle que la montmorillonite non organo-modifiée ou tout simplement un matériau sans charge tel que décrit dans l'exemple 1. Bien que le borate de zinc soit bien connu pour ses propriétés de retardateur de flamme, son effet ne se marque pas de manière très importante, ce qui s'explique par un faible taux de chargement utilisé (20%). Les propriétés anti-feu mesurées selon les mêmes nor-

mes que précedemment montrent également une amélioration, ainsi que l'attestent les résultats suivants :

Temps d'inflammation : 9 s
Hauteur maximale de la flamme pendant 20 sec suivant les 20 premières sec : 3 cm

Le repère n'est pas atteint.
L'amélioration se situe essentiellement au niveau de la hauteur maximale de la flamme.

**Exemple 6 : imprégnation avec le borate de zinc et la montmorillonite organomodifiée par des cations (2 éthyl-hexyl)diméthyl(suif hydrogéné)ammonium**

- Préparation de P :

Dans un bécher, on dissout 200 g de polycaprolactone diol de masse moléculaire 4 000 dans 200 g de dichlorométhane. On ajoute à cette solution 43,54 g de borate de zinc (microcharge), soit 20% en poids de charge par rapport au total de résine polycaprolactone, ainsi que 10,89 g de montmorillonite organo-modifiée par des cations (2 éthyl-hexyl)diméthyl(suif hydrogéné) ammonium, soit 10% en poids par rapport au total final de résine caprolactone et 0,13 g de DABCO.

- Préparation de PP :

Dans un bécher, on mélange 50 g de polycaprolactone diol de masse moléculaire 4 000 avec 50 g d'hexaméthylène diisocyanate dans 100 g de dichlorométhane.

- Mélange de P et PP

Dans le bécher contenant P, on ajoute 35,11 g de la solution PP. On verse le tout dans un récipient en aluminium permettant l'imprégnation du coton par trempes successives. On place ensuite ces échantillons de coton imprégné dans une étuve chauffée selon une rampe de température allant de la température ambiante à 150°C (voir exemples précédents). Le produit final est obtenu dès retour à la température ambiante.

Pour ce type d'échantillon, le degré d'imprégnation moyen (DRC) est de 82%. Les masses moléculaires moyennes en nombre et en masse sont également déterminées par analyse chromatographique par perméation de gel. Les résultats suivants ont été obtenus :
Mn = 19 500, Mw = 34 600, Mw/Mn = 1,77. L'analyse calorimétrique différentielle des températures caractéristiques donne la température de transition vitreuse et la température de fusion. Ces deux températures sont :
Tg = -64,79°C, Tm = 51,11°C.
Au niveau des propriétés mécaniques, on obtient pour les tests de traction les résultats suivants :

Module d'élasticité : $E_t$ = 81,26 N/mm
Charge maximale : $\sigma_c$ = 531,39 N

En ce qui concerne les tests de compression, on obtient :

Module de compression : $E_c$ (pente) = 45,86 N/mm
Charge pour une compression de 22,5 mm : $\sigma_c$ = 390,04 N

Dans cet exemple, on a ajouté à la résine non seulement des nanocharges, mais également des microcharges dont la taille typique est comprise entre 1 et 20 $\mu$m.
Ce mélange de nanocharges et de microcharges provoque une augmentation du module d'élasticité tout en modulant la charge maximale supportée au niveau de la traction ($\sigma_c$ intermédiaire aux compositions des exemples 1 et 6). Par contre, au niveau de la compression, la charge nécessaire pour une compression de 22,5 mm est une des plus importantes de tous les exemples décrits.
De plus, le matériau présente un aspect beaucoup plus lisse au toucher, la rugosité de surface étant nettement améliorée.
Outre ces améliorations, les propriétés anti-feu conférées au matériau par ce type de mélange de charges, sont excellentes comme en attestent les résultats suivants :

Temps d'inflammation : 13 s
Hauteur maximale de la flamme pendant 20 sec suivant les 20 premières sec : 2 cm

Le repère n'est pas atteint.
L'amélioration se situe essentiellement au niveau de la hauteur maximale de la flamme.
A nouveau, le matériau ne forme pas de gouttes enflammées (comportement similaire aux exemples 3 et 4), cette caractéristique étant attribuée à la formation de nanocomposites.

**Revendications**

1. Tissu de support flexible imprégné d'un matériau nanocomposite, formé à partir de résine de polyuréthane thermoplastique semi-cristalline et de précurseurs de nanocharges, **caractérisé en ce que** lesdites nanocharges présentent des couches silicates organo-modifiées en surface et sont dispersées dans ladite résine par intercalation et/ou exfoliation des différentes couches, lesdites nanocharges constituant des ponts de réticulation physique entre les chaînes polymères de ladite résine thermoplastique.

2. Tissu imprégné selon la revendication 1, **caractérisé en ce que** ladite résine thermoplastique est obtenue par mélange en présence d'un catalyseur d'une première solution organique de polymère et d'une seconde solution organique de prépolymère, le prépolymère (PP) étant un polymère diisocyanate obtenu à partir de la réaction d'un oligomère bifonctionnel avec un excès molaire de réactif diisocyanate et le polymère (P) étant un polymère bifonctionnel allongeur de chaîne.

3. Tissu imprégné selon la revendication 2, **caractérisé en ce que** ledit oligomère bifonctionnel comporte des fonctions terminales sélectionnées parmi le groupe constitué par -OH, $-NH_2$, -COOH et -SH, de préférence des diols de faible masse moléculaire.

4. Tissu imprégné selon la revendication 3, **caractérisé en ce que** ledit oligomère bifonctionnel est une polycaprolactone diol de masse moléculaire moyenne en nombre comprise entre 1000 et 10000, et de préférence entre 2000 et 6000.

5. Tissu imprégné selon l'une quelconque des revendications 2 à 4, **caractérisé en ce que** le réactif diisocyanate est sélectionné parmi le groupe des diisocyanates aromatiques, aliphatiques et cycloaliphatiques et est de préférence l'hexaméthylène diisocyanate ou le 4,4'-dicyclohexylméthane diisocyanate.

6. Tissu imprégné selon l'une quelconque des revendications 2 à 5, **caractérisé en ce que** le polymère bifonctionnel allongeur de chaîne est une polycaprolactone de faible masse moléculaire.

7. Tissu imprégné selon l'une quelconque des revendications 2 à 6, **caractérisé en ce que** le catalyseur est une amine tertiaire ou un composé à base d'étain, de préférence le dilaurate de dibutyl-étain.

8. Matériau nanocomposite selon l'une quelconque des revendications précédentes, **caractérisé en ce que** les nanocharges à couches silicates organomodifiées en surface présentent une substitution des ions sodium par des ions ammonium porteurs de chaînes alkyles, de préférence l'octadécylammonium quaternisé, le diméthyldioctadécylammonium, le (2 éthyl-hexyl)diméthyl(suif hydrogéné)ammonium, le (2-hydroxyéthyl)méthyl(suif hydrogéné)ammonium.

9. Tissu imprégné selon la revendication 8, **caractérisé en ce que** lesdites nanocharges sont constituées de montmorillonite organo-modifiée par des cations (2 éthylhexyl)diméthyl(suif hydrogéné)ammonium ou (2-hydroxyéthyl)méthyl(suif hydrogéné)ammonium ou un mélange des deux.

10. Tissu imprégné selon l'une quelconque des revendications précédentes, **caractérisé en ce que** le taux de chargement en nanocharges est compris entre 0,1 et 20% en poids, de préférence entre 2 et 15% en poids.

11. Tissu imprégné selon l'une quelconque des revendications précédentes, **caractérisé en ce qu'**il contient également des microcharges, de préférence le borate de zinc, le dihydroxyde de magnésium ou le trihydrate d'aluminium.

12. Tissu imprégné selon la revendication 11, **caractérisé en ce que** le taux de chargement en microcharges est compris entre 5 et 50% en poids, de préférence entre 10 et 30% en poids.

13. Tissu imprégné selon l'une quelconque des revendications précédentes, **caractérisé en ce qu'**il présente des propriétés anti-feu qualitativement améliorées par rapport à un matériau similaire ne comportant pas de nanocharges organo-modifiées ou comportant des nanocharges non organo-modifiées.

14. Tissu imprégné selon la revendication 13, **caractérisé en ce que** ledit matériau se consume sans formation de gouttes incandescentes lorsqu'il est mis au contact d'une flamme.

15. Tissu imprégné selon l'une quelconque des revendications 8 à 14, **caractérisé en ce que** lesdites nanocharges, organomodifiées par des ions ammonium porteurs de fonctions capables de réagir avec la fonction isocyanate, de préférence le cation (2-hydroxyéthyl)méthyl(suif hydrogéné)ammonium, réagissent en surface avec des chaînes polyuréthane par les extrémités isocyanates desdites chaînes et pontent ainsi lesdites chaînes entre elles.

16. Structure orthopédique comprenant un tissu de support flexible imprégné avec le matériau nanocomposite selon l'une quelconque des revendications précédentes, **caractérisée en ce que** ledit tissu de support est un tissu flexible à larges mailles, de préférence tissé ou tricoté, en matière cellulosique, de préférence en coton, ou synthétique, de préférence en acrylate ou nylon, et dont la plus petite dimension des ouvertures est comprise entre 0,142 et 1,613 $cm^2$, de préférence égale à 0,323 $cm^2$.

17. Procédé, de préférence pour l'obtention d'une structure orthopédique, comprenant l'imprégnation d'un tissu de support flexible, avec une composition comprenant un prépolymère (PP) comportant deux groupes terminaux isocyanante, un polymère bifonctionnel (P) allongeur de chaîne, des précurseurs de nanocharges organo-modifiées, au moins un catalyseur et un solvant, **caractérisé par** les étapes suivantes :

> - la préparation d'une solution de prépolymère (PP) diisocyanante dans un solvant organique, à partir de la réaction d'un oligomère bifonctionnel avec un excès molaire de réactif diisocyanate ;
> - la préparation d'une solution organique de polymère bifonctionnel (P) allongeur de chaîne, contenant également lesdites nanocharges organo-modifiées et un catalyseur ;
> - l'imprégnation dudit tissu de support flexible par trempes successives dans un mélange des deux dites solutions ;
> - l'élimination du solvant par mise en température selon une rampe de chauffe allant de la température ambiante jusqu'à 150°C ;
> - le retour à la température ambiante pour l'obtention d'un produit final de ladite structure orthopédique.

18. Utilisation du tissu de support flexible imprégné d'un matériau nanocomposite selon l'une quelconque des revendications 1 à 15 comme matériau résistant au feu dans le domaine des décors et parements théâtraux et architecturaux.

**Claims**

1. Flexible support fabric impregnated with a nanocomposite material, formed from semi-crystalline thermoplastic polyurethane resin and from precursors of nanocharges, **characterised in that** said nanocharges have organomodified silicate layers in their surfaces and are dispersed in said resin by insertion and/or exfoliation of the various layers, said nanocharges forming physical reticulation bridges between the polymer chains of said thermoplastic resin.

2. Impregnated fabric according to Claim 1, **characterised in that** said thermoplastic resin is obtained by mixing, in the presence of a catalyst, a first organic solution of polymer and a second organic solution of prepolymer, the prepolymer (PP) being a diisocyanite polymer obtained from the reaction of a bifunctional oligomer with a molar excess of diisocyanite reagent and the polymer (P) being a chain-extending bifunctional polymer.

3. Impregnated fabric according to Claim 2, **characterised in that** said bifunctional oligomer comprises terminal functions selected from the group of -OH,

$-NH_2$, -COOH and -SH, preferably diols of low molecular weight.

4. Impregnated fabric according to Claim 3, **characterised in that** said bifunctional oligomer is a diol polycaprolactone of average molecular weight by number between 1,000 and 10,000, and preferably between 2,000 and 6,000.

5. Impregnated fabric according to any one of Claims 2 to 4, **characterised in that** the diisocyanate reagent is selected from the group of aromatic, aliphatic and cycloaliphatic diisocyanates, and is preferably hexamethylene diisocyanate or 4,4'-dicyclohexylmethane diisocyanate.

6. Impregnated fabric according to any one of Claims 2 to 5, **characterised in that** the chain-extending bifunctional polymer is a polycaprolactone of low molecular weight.

7. Impregnated fabric according to any one of Claims 2 to 6, **characterised in that** the catalyst is a tertiary amine or a tin-based compound, preferably dibutyltin dilaurate.

8. Nanocomposite material according to any one of the preceding claims, **characterised in that** the nanocharges with organomodified silicate layers on the surface have sodium ions substituted by ammonium ions carrying alkyl chains, preferably quaternised octadecylammonium, dimethyldioctadecyclammonium, (2 ethylhexyl)dimethyl(hydrogenated tallow)ammonium, (2-hydroxyethyl)methyl(hydrogenated tallow)ammonium.

9. Impregnated fabric according to Claim 8, **characterised in that** said nanocharges are made of montmorillonite organomodified by cations (2 ethylhexyl)-dimethyl(hydrogenated tallow)ammonium or (2-hydroxyethyl)-methyl(hydrogenated tallow)ammonium or a mixture of both.

10. Impregnated fabric according to any one of the preceding claims, **characterised in that** the level of charge with nanocharges is between 0.1 and 20% by weight, preferably between 2 and 15% by weight.

11. Impregnated fabric according to any one of the preceding claims, **characterised in that** it also comprises microcharges, preferably zinc borate, magnesium dihydroxide or aluminium trihydrate.

12. Impregnated fabric according to Claim 11, **characterised in that** the charge level of microcharges is between 5 and 50% by weight, preferably between 10 and 30% by weight.

**13.** Impregnated fabric according to any one of the preceding claims, **characterised in that** it has fireproof properties that are qualitatively improved relative to a similar material that does not comprise organomodified nanocharges or that comprises nanocharges that are not organomodified.

**14.** Impregnated fabric according to Claim 13, **characterised in that** said material is consumed without forming incandescent drops when it is brought into contact with a flame.

**15.** Impregnated fabric according to any one of Claims 8 to 14, **characterised in that** said nanocharges, organomodified by ammonium ions carrying functions capable of reacting with the isocyanate function, preferably the cation (2-hydroxyethyl)methyl (hydrogenated tallow)-ammonium, react on the surface with polyurethane chains by the isocyanate ends of said chains and thus bridge said chains to each other.

**16.** Orthopaedic structure comprising a flexible support fabric impregnated with the nanocomposite material according to any one of the preceding claims, **characterised in that** said support fabric is a large-mesh flexible fabric, preferably woven or knitted, in a cellulose material, preferably cotton, or synthetic, preferably in acrylate or nylon, and whose smallest opening dimension is between 0.142 and 1.613cm$^2$, preferably equal to 0.323cm$^2$.

**17.** Method, preferably for obtaining an orthopaedic structure, comprising the impregnation of a flexible support fabric, with a composition comprising a prepolymer (PP) with two isocyanate terminal groups, a chain-extending bifunctional polymer (P), organomodified nanocharge precursors, at least one catalyst and one solvent, **characterised by** the following stages:

- preparation of a diisocyanate prepolymer (PP) solution in an organic solvent, based from the reaction of a bifunctional oligomer with a molar excess of diisocyanate reagent;
- preparation of an organic solution of chain-extending bifunctional polymer (P) also comprising said organomodified nanocharges and a catalyst;
- impregnation of said flexible support fabric by successive baths in a mixture of said two solutions;
- removal of the solvent by heating up according to a heating gradient rising from the ambient temperature to 150°C;
- return to ambient temperature to obtain a final product of said orthopaedic structure.

**18.** Use of the flexible support fabric impregnated with a nanocomposite material according to any one of Claims 1 to 15 as a fire-resistant material in the area of scenery and ornamentation for theatres and architecture.

**Patentansprüche**

**1.** Flexibles Stützgewebe, das mit einem Nanoverbundmaterial imprägniert ist, das aus teilkristallinem, thermoplastischen Polyurethanharz und Zwischenstoffen von Nanoladungen gebildet wird, **dadurch gekennzeichnet, dass** die besagten Nanoladungen organisch modifizierte Silikatschichten an der Oberfläche aufweisen und in dem besagten Harz durch Verschachtelung und/oder Abblätterung der verschiedenen Schichten verstreut sind, wobei die besagten Nanoladungen physikalische Vernetzungsbrücken zwischen den Polymerketten des besagten thermoplastischen Harzes bilden.

**2.** Imprägniertes Gewebe nach Anspruch 1, **dadurch gekennzeichnet, dass** das besagte thermoplastische Harz durch Mischung einer ersten organischen Polymerlösung und einer zweiten organischen Prepolymerlösung mit vorhandenem Katalysator hergestellt wird, wobei das Prepolymer (PP) ein Diisocyanat-Polymer ist, das durch die Reaktion eines bifunktionalen Oligomers mit einem molaren Überschuss von Diisocyanat-Reagens hergestellt wird, und das Polymer (P) ein bifunktionales Kettenverlängerungspolymer ist.

**3.** Imprägniertes Gewebe nach Anspruch 2, **dadurch gekennzeichnet, dass** das besagte bifunktionale Oligomer Endgruppen aufweist, die aus der Gruppe -OH, NH$_2$, - COOH und -SH ausgewählt werden, vorzugsweise Diole mit geringer Molekularmasse.

**4.** Imprägniertes Gewebe nach Anspruch 3, **dadurch gekennzeichnet, dass** das besagte bifunktionale Oligomer ein Polycaprolacton-Diol mit einer mittleren Molekularmasse in der Zahl zwischen 1000 und 10000 ist, vorzugsweise zwischen 2000 und 6000.

**5.** Imprägniertes Gewebe nach einem der Ansprüche 2-4, **dadurch gekennzeichnet, dass** das Diisocyanat-Reagens aus der Gruppe der aromatischen, aliphatischen und cycloaliphatischen Diisocyanate ausgewählt wird, vorzugsweise Hexamethylen-Diisocyanat oder 4,4'-Dicyclohexylmethan-Diisocyanat.

**6.** Imprägniertes Gewebe nach einem der Ansprüche 2-5, **dadurch gekennzeichnet, dass** das bifunktionale Kettenverlängerungspolymer ein Polycaprolacton geringer Molekularmasse ist.

**7.** Imprägniertes Gewebe nach einem der Ansprüche 2-6, **dadurch gekennzeichnet, dass** der Katalysator ein tertiäres Amin oder eine Verbindung auf Zinnbasis ist, vorzugsweise Dibutylzinndilaurat.

**8.** Nanoverbundmaterial nach einem der vorstehenden Ansprüche, **dadurch gekennzeichnet, dass** die Nanoladungen mit organisch modifizierten Silikatschichten an der Oberfläche einen Austausch der Natriumionen durch Ammoniumionen, die Träger von Alkylketten sind, aufweisen, vorzugsweise quaternisiertes Octadecylammonium, Dimethyldioctadecylammonium, (2 Ethylhexyl)Dimethyl (hydrierter Talg)Ammonium, (2 Hydroxethyl)Methyl(hydrierter Talg) Ammonium.

**9.** Imprägniertes Gewebe nach Anspruch 8, **dadurch gekennzeichnet, dass** die besagten Nanoladungen aus Montmorillonit bestehen, das durch Kationen (2 Ethylhexyl)Dimethyl(hydrierter Talg)Ammonium oder (2 Hydroxethyl)Methyl(hydrierter Talg) Ammonium oder einer Mischung von beiden organisch modifiziert ist.

**10.** Imprägniertes Gewebe nach einem der vorstehenden Ansprüche, **dadurch gekennzeichnet, dass** die Nanoladungsrate zwischen 0,1 und 20 Gewichts-% beträgt, vorzugsweise zwischen 2 und 15 Gewichts-%.

**11.** Imprägniertes Gewebe nach einem der vorstehenden Ansprüche, **dadurch gekennzeichnet, dass** es ebenfalls Mikroladungen enthält, vorzugsweise Zinkborat, Magnesiumdihydroxid oder Aluminiumtrihydrat.

**12.** Imprägniertes Gewebe nach Anspruch 11, **dadurch gekennzeichnet, dass** die Mikroladungsrate zwischen 5 und 50 Gewichts-% beträgt, vorzugsweise zwischen 10 und 30 Gewichts-%.

**13.** Imprägniertes Gewebe nach einem der vorstehenden Ansprüche, **dadurch gekennzeichnet, dass** es flammwidrige Eigenschaften aufweist, die im Vergleich zu einem ähnlichen Material, das keine organisch modifizierten Nanoladungen oder nicht organisch modifizierte Nanoladungen aufweist, qualitativ verbessert sind.

**14.** Imprägniertes Gewebe nach Anspruch 13, **dadurch gekennzeichnet, dass** das besagte Material ohne Bildung von glühenden Tropfen verbrennt, wenn es mit einer Flamme in Berührung kommt.

**15.** Imprägniertes Gewebe nach einem der Ansprüche 8 bis 14, **dadurch gekennzeichnet, dass** die besagten Nanoladungen, die durch Ammoniumionen organisch modifiziert sind, die Träger von Gruppen sind, die fähig sind, mit der Isocyanat-Gruppe zu reagieren, vorzugsweise das Kation (2 Hydroxethyl)Methyl(hydrierter Talg)Ammonium, an der Oberfläche mit Polyurethanketten durch die Isocyanatenden der besagten Ketten reagieren und so die besagten Ketten miteinander verbinden.

**16.** Orthopädische Struktur, bestehend aus einem flexiblen Stützgewebe, das mit einem Nanoverbundmaterial nach einem der vorstehenden Ansprüche imprägniert ist, **dadurch gekennzeichnet, dass** das besagte Stützgewebe ein flexibles, weitmaschiges Gewebe ist, vorzugsweise gewebt oder gestrickt, aus Zellulosematerial, vorzugsweise Baumwolle, oder aus Synthetikmaterial, vorzugsweise Acrylat oder Nylon, dessen kleinste Maschenweite zwischen 0,142 und 1,613 cm$^2$ beträgt, vorzugsweise 0,323 cm$^2$.

**17.** Verfahren, vorzugsweise zur Herstellung einer orthopädischen Struktur, das die Imprägnierung eines flexiblen Stützgewebes umfasst, mit einer Zusammensetzung, die ein Prepolymer (PP) umfasst, das zwei IsocyanatEndgruppen beinhaltet, ein bifunktionales Kettenverlängerungspolymer (P), Zwischenstoffe von organisch modifizierten Nanoladungen, mindestens einen Katalysator und ein Lösungsmittel, durch die folgenden Phasen **gekennzeichnet**:

- Zubereitung einer Prepolymer(PP)-Diisocyanat-Lösung in einem organischen Lösungsmittel durch die Reaktion eines bifunktionalen Oligomers mit einem molaren Überschuss von Diisocyanat-Reagens
- Zubereitung einer organischen bifunktionalen Kettenverlängerungspolymer (P)-Lösung, die ebenfalls die besagten organisch modifizierten Nanoladungen und einen Katalysator enthält,
- Imprägnierung des besagten flexiblen Stützgewebes durch aufeinander folgendes Eintauchen in eine Mischung der beiden besagten Lösungen,
- Entfernen des Lösungsmittels durch Erwärmung von Umgebungstemperatur bis auf 150°C,
- Rückkehr zur Umgebungstemperatur, um ein Endprodukt der besagten orthopädischen Struktur zu erhalten.

**18.** Verwendung des flexiblen Stützgewebes, das mit einem Nanoverbundmaterial nach einem der Ansprüche 1 bis 15 imprägniert ist, als flammwidriges Material im Bereich der Theater- und Architekturdekore und - verkleidungen.

FIG. 1a

FIG. 1b

FIG. 2a

FIG. 2b